# NEUE EUROPÄISCHE PATENTSCHRIFT

(11) **EP 0 669 323 B2**
(45) Veröffentlichungstag und Bekanntmachung des Hinweises auf die Entscheidung über den Einspruch: **07.04.2004**
(45) Hinweis auf die Patenterteilung: 09.05.2001
(21) Anmeldenummer: 95101906.6
(22) Anmeldetag: 13.02.1995
(51) Int. Cl.: C07D 235/20, A61K 7/42, C07D 277/66, C07D 263/62, C07D 263/64

(54) **KOSMETISCHE UND DERMATOLOGISCHE ZUBEREITUNGEN, ENTHALTEND PHENYLEN-1,4-BISBENZIMIDIAZOLESULFONSÄUREN**
COSMETIC AND DERMATOLOGICAL PREPARATIONS, CONTAINING PHENYLENE-1,4-BISBENZIMIDAZOLE SULFONIC ACIDS
COMPOSITIONS COSMETIQUES ET DERMATOLOGIQUES CONTENANT D' ACIDES SULFONIQUES DE PHENYLENE-1,4-BISBENZIMIDAZOLE

(30) Priorität: 24.02.1994 DE 4406024; 22.03.1994 DE 4409689
(43) Veröffentlichungstag der Anmeldung: 30.08.1995
(73) Patentinhaber: HAARMANN & REIMER GMBH, 37601 Holzminden (DE)
(72) Erfinder: Pelzer, Dr. Ralf, D-37699 Fürstenberg (DE); Langner, Roland, D-37639 Bevern (DE); Surburg, Dr. Horst, D-37603 Holzminden (DE); Sommer, Dr. Horst, D-37603 Holzminden (DE); Krempel, Alfred, D-37603 Holzminden (DE); Hopp, Dr. Rudolf, D-37603 Holzminden (DE)
(74) Vertreter: Eisenführ, Speiser & Partner

(56) Entgegenhaltungen:
- WO-A-93/15061
- WO-A-93/15712
- CH-A- 350 763
- DE-A- 2 550 876
- DE-B- 1 282 855
- GB-A- 1 198 632
- US-A- 2 463 264
- US-A- 3 808 005
- Roempp Chemie Lexikon, 6 Aufl., Band IV, 1966, 6258-6259 und 6262-6263

## Beschreibung

Die Erfindung betrifft kosmetische und dermatologische Zubereitungen, die Verwendung von bestimmten Phenylen-1,4-bisbenzimidazolsulfonsäuren als UV-Absorber in Sonnenschutzmitteln, außerdem neue Phenylen-1,4-bisbenzimidazolsulfonsäuren und ein Verfahren zur Herstellung kosmetischer und dermatologischer Zubereitungen, in dem entsprechende difunktionelle Aniline mit reakrtiven Carbonsäurederivaten umgesetzt werden.

UV-Strahlen werden je nach Wellenlänge als UV-A-Strahlen (320-400 nm, UV-A-I: 340-400 nm, UV-A-II: 320-340 nm) oder UV-B-Strahlen (280-320 nm) bezeichnet. Ganz allgemein gilt: Die schädigende Wirkung der UV-Strahlen auf die menschliche Haut steigt mit sinkender Wellenlänge und steigender Dauer der Exposition.

So können UV-Strahlen Hautschädigungen hervorrufen, wobei die UV-B-Strahlung einen Sonnenbrand (Erythem) bis hin zu schwersten Hautverbrennungen verursachen kann. Sehr häufige und ungeschützte Bestrahlung der Haut mit Sonnenlicht führt auch zu einem Verlust der Hautelastizität und zu vermehrter Faltenbildung, insgesamt zu frühzeitiger Alterung der Haut In extremen Fällen können krankhafte Hautveränderungen bis hin zum Hautkrebs auftreten.

Die UV-A-Strahlung bewirkt eine rasche, schwache Direktpigmentierung der Haut. UV-A-Strahlen dringen in tiefere Hautschichten ein und können dort den Alterungsprozeß der Haut beschleunigen. Die kürzerwellige UV-A-II-Strahlung unterstützt die Bildung von Sonnenbrand. Weiterhin kann die UV-A-Strahlung phototoxische oder photoallergische Hautreaktionen auslösen. Es existieren gesicherte Zusammenhänge zwischen UV-A-Exposition und erhöhtem Hautkrebsrisiko.

Entsprechend der Lage ihrer Absorptionsmaxima werden UV-Absorber für kosmetische und dermatologische Präparate in UV-A- und UV-B-Absorber eingeteilt.

Es gibt eine größere Anzahl sicherer und effektiver UV-B-Absorber, wie z.B. p-Methoxyzimtsäureisooctylester, p-Methoxyzimtsäureisoamylester, Phenylbenzimidazol-Nasulfonat und 3-(4'-Methylbenzyliden)-campher.

Die Anzahl für den Schutz der menschlichen Haut geeigneter UV-A-Absorber ist jedoch nicht nur sehr begrenzt, sondern diese Absorber sind auch mit gravierenden Nachteilen behaftet:

Dibenzoylmethanderivate (4-t-Butyl-methoxy-dibenzoylmethane und 4-Isopropyl-dibenzoylmethane) sind nicht sehr lichtstabil, d.h. ihre UV-A-Schutzleistung läßt während der Anwendung rasch nach. Ferner haben sie nur eine begrenzte Löslichkeit in kosmetischen Ölen, was zu Problemen in der Formulierung kosmetischer Zubereitungen führen kann. Das Absorptionsmaximum liegt in dem weniger gefährlichen UV-A-I-Bereich. Außerdem können Dibenzoylmethanderivat-haltige Sonnenschutzprodukte auf Textilien extrem schwer auswaschbare Flecken hinterlassen. Bekannt ist auch, daß Dibenzoylmethane Photosensibilisierungen der Haut begünstigen können.

Benzophenone sind UV-B- und UV-A-Breitbandabsorber und besitzen im kurzwelligen UV-A-II-Bereich nur eine verhältnismäßig geringe Absorption. Ihre Löslichkeit in kosmetischen Ölen ist ebenfalls begrenzt.

Menthyl-O-aminobenzoat besitzt eine nur sehr schwache Absorption im UV-A-Bereich.

Terephthalyliden-dibornansulfonsäure besitzt ein Absorptionsmaximum im UV-A-I-Bereich bei 345 nm.

Gesucht werden UV-A-Absorber, die ein Absorptionsmaximum im UV-A-II-Bereich aufweisen und durch eine starke Absorption vor den gefährlichen, kurzwelligen UV-A-Strahlen schützen. Daneben sollen diese UV-A-Absorber noch folgende Kriterien erfüllen: ausgezeichnete Lichtbeständigkeit, toxikologische und dermatologische Unbedenklichkeit, ausgezeichnete Thermostabilität, sehr gute Löslichkeit in kosmetischen Lösungsmitteln (Öle, Wasser, Glykole, Alkohol usw.), Verträglichkeit mit kosmetischen Grundstoffen, pH-stabil (4-9), problemlose Verarbeitbarkeit in kosmetischen Formulierungen und Stabilität unter Anwendungsbedingungen, Verträglichkeit mit Verpackungsmaterialien, keine Färbung von Textilien, mindestens jedoch: Flecken auf Textilien müssen problemlos auswaschbar sein; möglichst Farblosigkeit und Geruchsneutralität, Klebfreiheit und geringe Flüchtigkeit

In DE-B 676103 werden das Natriumsalz der Phenylbenzimidazolsulfonsäure (Absorptionsmaximum: 316 nm) und ähnliche Verbindungen als UV-Absorber in Sonnenschutzmitteln für die menschliche Haut empfohlen. Die beschriebenen Verbindungen besitzen aber nicht die gewünschte Lichtstabilität bzw. das gewünschte Absorptionsmaximum.

In DE-B 1 282 855 werden Phenylen-1,3-bis-benzimidazol-5,5'-disulfonsäuren beschrieben, die im UV-Bereich absorbieren. Diese Verbindungen absorbieren jedoch nur unzureichend im UV-A-Bereich und sind daher für effektive Sonnenschutzmittel nicht geeignet.

Die WO 93/150 61 offenbart Phenylen-1,4-bisbenzimidazol-5,5'-disulfonsäure und ihre Verwendung als UV-A-Filter.

Es wurden kosmetische und dermatologische Zubereitungen gefunden, die bislang nicht bekannte Phenylen-1,4-bisbenzimidazolsulfonsäuren als UV-A-Absorber enthalten.

Phenylen-1,4-bisbenzimidazolsulfonsäuren absorbieren überraschenderweise in starkem Maß UV-A-Strahlen und sind besonders für die erfindungsgemäßen kosmetischen und dermatologischen Zubereitungen geeignet.

Erfindungsgemäße kosmetische und dermatologische Zubereitung enthalten die bislang nicht bekannten Verbindungen

Phenylen-1,4-bisbenzimidazol-5,5',7-trisulfonsäure bzw. deren Salze oder Phenylen-1,4-bisbenzimidazol-5,5',7,7'-tetrasulfonsäure bzw. deren Salze als UV-Absorber.

Gegenstand der Erfindung ist ebenfalls die Verwendung der besagten Phenylen-1,4-bisbenzimidazolsulfonsäuren als UV-A-Absorber in kosmetischen und dermatologischen Zubereitungen.

Mit den besagten Phenylen-1,4-bisbenzimidazolsulfonsäuren wurden überraschenderweise Verbindungen gefunden, die nicht nur aufgrund ihres Absorptionsmaximums im UV-A-II-Bereich die gefährlichen UV-Strahlen absorbieren, sondern auch eine ausgezeichnete Lichtstabilität besitzen. Im Vergleich zu bekannten Verbindungen steigt die Wasserlöslichkeit nach Neutralisation mit üblichen Basen (z.B. Natriumhydroxid, Kaliumhydroxid, Triethanolamin, Monoethanolamin, Tetrahydroxypropylethylendiamin, Tris(hydroxymethyl)-aminomethan usw.) beträchtlich, was zu einer unproblematischen Einarbeitung in kosmetische Grundlagen führt. Hervorzuheben ist, daß auch kosmetische oder dermatologische Zubereitungen mit einem niedrigen pH-Wert (bis pH 4) stabil formuliert werden können, ohne daß eine Auskristallisation eintritt. Zubereitungen mit einem hohen UV-Absorber-Gehalt (beispielsweise bis zu 20 Gew.-%) sind möglich.

Es fällt auf, daß durch die Zugabe einer Base bis zum Erreichen eines pH-Wertes über 10 eine Verschiebung des Absorptionsmaximums zum längerwelligen Bereich hin (beispielsweise von 335 nach 355 nm) eintritt. Es ist also möglich, bei Bedarf diese Schutzfunktion der erfindungsgemäß zu verwendenden Verbindungen in den UV-A-I-Bereich zu verschieben.

Bestimmte Phenylen-1,4-bisbenzimidazolsulfonsäuren sind bereits bekannt.

Die WO 93/15061 wurde bereits genannt.

Pharmazeutisch verwendbare Benzazolylverbindungen mit lediglich einer Benzazolylgruppe sind beispielsweise aus der DE-A 35 33 308 beschrieben. Sie werden u.a. gegen Haarausfall, zur Bekämpfung von Hautkrankheiten und des fettigen Aussehens der Haut, zur Bekämpfung von trockener Haut und zur Behandlung der nachteiligen Wirkungen der Sonne empfohlen. Eine prophylaktische Verwendung gegen den schädigenden Einfluß von UV-Strahlen auf die menschliche Haut wird jedoch nicht erwähnt.

In der US 2 463 264 wird beispielsweise die Verwendung einiger Benzazoylverbindungen als optische Aufheller beschrieben. Die Verwendung gegen den schädigenden Einfluß von UV-Strahlen auf die menschliche Haut wird weder erwähnt noch nahegelegt.
Phenylen-1,4-bisbenzimidazol-5,5',7-trisulfonsäure und ihre Salze und
Phenylen-1,4-bisbenzimidazol-5,5',7,7'-tetrasulfonsäure und ihre Salze sind neu.

Die erfindungsgemäßen Phenylen-1,4-bisbenzimidazolsulfonsäuren können nach bekannten Verfahren oder analog zu diesen bekannten Verfahren hergestellt werden. Eine der möglichen Verfahrensvarianten ist die Umsetzung von Carbonsäurederivaten mit o-Phenylendiamin bei höheren Temperaturen in hochsiedenden Aromaten, wie z.B. Diphenylether und/oder Biphenyl, oder in anorganischen Säuren wie Phosphorsäure oder Schwefelsäure. Sulfonsäuregruppen können entweder vor der Kondensation durch Sulfonierung der aromatischen Amine oder nach der Kondensation durch Behandlung mit Oleum, heißer Schwefelsäure oder Chlorsulfonsäure eingeführt werden. Nach einer anderen Verfahrensvariante werden Aldehyde mit den aromatischen Aminen unter oxidativen Bedingungen umgesetzt.

Nach einer bevorzugten Ausführungsform werden Kondensation und Sulfonierung in einem einzigen Schritt vollzogen: Chlorsulfonsäure ist gleichzeitig Reaktionsmedium und Reagenz.

Gegenstand der Erfindung ist auch ein Verfahren zur Herstellung der erfindungsgemäßen kosmetischen und dermatologischen Zubereitungen, dadurch gekennzeichnet, daß die Phenylen-1,4-bisbenzimidazolsulfonsäuren durch Umsetzung von o-Phenylendiamin und Terephthalsäure in Chlorsulfonsäure bei Temperaturen von 20 bis 190°C hergestellt und mit für diese Anwendung üblicherweise verwendeten Bestandteilen verarbeitet wird.

Die erfindungsgemäß zu verwendenden Phenylen-1,4-bisbenzimidazolsulfonsäuren und deren Salze können als UV-Absorber in kosmetischen oder dermatologischen Zubereitungen in Mengen verwendet werden, die den Durchtritt der UV-Strahlen durch den aufgetragenen Film der Zubereitung verhindern. Dies ist dann der Fall, wenn die kosmetischen bzw. dermatologischen Zubereitungen 0,5 bis 15, vorzugsweise 1 bis 10, insbesondere 3 bis 8 Gew.-%, bezogen auf das Gesamtgewicht der Zubereitung, der erfindungsgemäß zu verwendenden Verbindung enthalten.

Die Phenylen-1,4-bisbenzimidazolsulfonsäuren enthaltenden Zubereitungen können zum Schutz der Haut und der Haare - insbesondere durch Dauerwelle, Färbung und Bleichung bereits vorgeschädigten Haare - vor UV-Bestrahlung verwendet werden. Diese zum Schutz der Haut vor der UV-Strahlung dienenden kosmetischen oder dermatologischen Zubereitungen können in den üblicherweise verwendeten Anwendungsformen vorliegen, d.h. als Öl-in-Wasser- oder Wasser-in-Öl-Emulsion, als Milch, Lotion oder Creme, wäßrig oder wäßrig-alkoholisches Gel oder Lotion, Aerosol, Hydrodispersions-Gel (emulgatorfrei) oder jegliche andere übliche kosmetische oder dermatologische Zubereitung. Für den Schutz der Haare vor UV-Strahlen werden bevorzugt Zubereitungen als Shampoo, Spülung, Kur, Gel, Lotion, Spray oder Creme verwendet.

Die kosmetischen und dermatologischen Zubereitungen können die in diesen Mitteln üblicherweise verwendeten Bestandteile wie z.B. Emulgatoren, grenzflächenaktive Verbindungen, Lanolin, Vaseline, Wasser, Triglyceride von Fettsäuren, Polyethylenglykole, Fettalkohole, ethoxylierte Fettalkohole, Fettsäureester (z.B. Isopropylpalmitat, Isooctylstearat, Adipinsäurediisopropylester usw.), natürliche oder synthetische Öle oder Wachse, Pigmente (z.B. Titandioxid, Zinkoxid, Perlglanzpigmente, Farbpigmente), Verdickungsmittel (z.B. Hydroxyethylcellulose, Bentonit usw.), Konservierungsstoffe, Feuchtigkeitsmittel, Vitamine, Siliconöle, Glycerin, Ethylalkohol, Parfumöle enthalten.

Die erfindungsgemäß zu verwendenden Verbindungen können als einzige UV-Absorber in den entsprechenden Zubereitungen eingesetzt werden; man kann sie jedoch auch in Kombination mit anderen UV-Absorbern - insbesondere UV-B-Absorbern zur Erzielung einer UV-A+B-Breitbandabsorption oder mit schwach lichtstabilen Dibenzoylmethan-Derivaten (z.B. Butyl-methoxydibenzoyl-methan oder 4-isopropyl-dibenzoylmethan) zu deren Stabilisierung - einsetzen. Beispiele für solche Verbindungen umfassen
p-Aminobenzoesäure
p-Aminobenzoesäureethylester (25 Mol) ethoxyliert
p-Dimethylaminobenzoesäure-2-ethylhexylester
p-Aminobenzoesäureethylester (2 Mol) N-propoxyliert
p-Aminobenzoesäureglycerinester
Salicylsäure-homomenthylester
Salicylsäure-2-ethylhexylester
Triethanolaminsalicylat
4-Isopropylbenzylsalicylat
Anthranilsäurementhylester
Diisopropylzimtsäureethylester
p-Methoxyzimtsäure-2-ethylhexylester
Diisopropylzimtsäuremethylester
p-Methoxyzimtsäure-isoamylester
p-Methoxyzimtsäure-diethanolaminsalz
p-Methoxyzimtsäure-isopropylester
2-Ethylhexyl-2-cyano-3,3-diphenylacrylat
Ethyl-2-cyano-3,3-diphenylacrylat
2-Phenylbenzimidazolsulfonsäure und Salze
N,N,N-Trimethyl-4-(2-oxoborn-3-ylidenmethyl)-aniliniummethylsulfat
Tetraphthalyliden-dibornansulfonsäure und Salze
4-t-Butyl-4'-methoxy-benzoylmethan
β-imidazol-4(5)-acrylsäure (Urocaninsäure)
2-Hydroxy-4-methoxybenzophenon
2-Hydroxy-4-methoxybenzophenon-5-sulfonsäure
Dihydroxy-4-methoxybenzophenon
2,4-Dihydroxybenzophenon
Tetrahydroxybenzophenon
2,2'-Dihydroxy-4,4'-dimethoxybenzophenon
2-Hydroxy-4-n-octoxybenzophenon
2-Hydroxy-4-methoxy-4'-methylbenzophenon
α-(2-Oxoborn-3-yliden)-tolyl-4-sulfonsäure und Salze
3-(4'-Methylbenzyliden)-d,1-campher
3-Benzyliden-d,1-campher
4-Isopropyldibenzoylmethan
2,4,6-Trianilino-(p-carbo-2'-ethylhexyl-1'-oxy)-1,3,5-triazin
Besonders geeignete UV-B-Absorber sind
p-Methoxyzimtsäure-2-ethylhexylester,
p-Methoxyzimtsäure-isoamylester,
2-Phenylbenzimidazolsulfonsäure und
3-(4'-Methylbenzyliden)-d,1-campher.

Die in den nachfolgenden Beispielen angegebenen Teile sind Gewichtsteile, Prozentangaben beziehen sich jeweils auf das Gewicht.

### Beispiele

### Beispiel 1

Phenylen-1,4-bisbenzimidazol-5,5',7,7'-tetrasulfonsäure

108 Teile gereinigtes o-Phenylendiamin werden in 500 Teilen H₂SO₄ konz. eingetragen und 800 Teile Chlorsulfonsäure zugetropft. Das Gemisch wird 15 Min. bei 120°C erhitzt, wobei kontinuierlich HCl freigesetzt wird, auf 70°C abgekühlt und mit 83 Teilen Terephthalsäure versetzt. Das Reaktionsgemisch setzt weiter HCl frei und wird auf 180°C erhitzt. Nach einer Reaktionszeit von 30 Min. bei dieser Temperatur wird auf 80°C abgekühlt und auf 2500 Teile Eis gegeben. Das ausgefallene Kristallisat wird abfiltriert, in Natronlauge aufgenommen und mit Aktivkohle erhitzt. Nach dem Ausfällen mit H₂SO₄ und Trocknen des Kristallisats im Vakuum erhält man 299 Teile Produkt. Dieses Produkt enthält nach RP-HPLC (MeOH/H₂O) 99 % der Titelsubstanz sowie ca. 1 % der Trisulfonsäure. Nach NMR stehen die Sulfonsäuregruppierungen in meta-Stellung zueinander. (Zum Vergleich der NMR-Daten ist unbedingt der pH-Wert einzustellen, da die Shiftwerte der hier vorgestellten Verbindungen stark pH-abhängig sind.) Der Schmelzpunkt ist >280°C.
λmax₁ = 208; E^{1%}_{1cm} = 1142; λmax₂ = 256; E^{1%}_{1cm} = 280,2;λmax₃ = 335;
E^{1%}_{1cm} = 745.

### Beispiel 2

### Phenylen-1,4-bisbenzimidazol-5,5',7-trisulfonsäure

Folgt man der Darstellung in Beispiel 1, hält jedoch die Reaktionstemperatur unter 70°C, wird in der Hauptsache das trisulfonierte Produkt erhalten, das nach RP-HPLC-Isolierung durch NMR-Spektroskopie charakterisiert werden kann.
λmax₁=206;E^{1%}_{1cm}=650;λmax₂=254;E^{1%}_{1cm}=162;λmax₃=335;
E^{1%}_{1cm} = 756.

### Beispiel 3

### Phenylen-1,4-bisbenzimidazol-5,5'-disulfonsäure (nicht erfindungsgemäß)

550 Teile konzentrierte Schwefelsäure (96 %ig) werden vorgelegt und sukzessive 108 Teile o-Phenylendiamin eingetragen. Die Reaktion ist exotherm und das Gemisch erhitzt sich auf 95-100°C. Bei dieser Temperatur werden 83 Teile Terephthalsäure eingetragen und das Gesamtgemisch 5 h bei 200°C erhitzt. Nach Abkühlung auf 100-120°C wird auf 1000 Teile Eis gegossen und 1 h gerührt. Der grüne bis braune Niederschlag wird abfiltriert, in NaOH aufgenommen und mit 20 Teile Aktivkohle 1 h unter Rückfluß erhitzt. Nach Filtration wird der pH-Wert mit H₂SO₄ auf 1,5 bis 2 eingestellt und der helle Niederschlag abfiltriert. Das Produkt wird mit Methanol aufgeschlämmt, abfiltriert und getrocknet.
λmax₁ = 218; E^{1%}_{1 cm} = 1057; λmax₂ = 256; E^{1 %}_{1 cm} = 424; λmax₃ = 351;
E^{1 %}_{1 cm} = 1289.

### Beispiel 4

### Sonnenschutzmilch (O/W)

Von Phenylen-1,4-bisbenzimidazol-5,5',7,7'-tetrasulfonsäure wurde eine 20 %ige Vorlösung, neutralisiert mit Natriumhydroxid, hergestellt. Von dieser Vorlösung wurden 20 % eingesetzt, was einem Aktivgehalt von 4,00 % UV-A-Absorber in der Endformulierung entspricht.

| | Bestandteile | % |
|---|---|---|
| A) | Cutina FS 45 | 2,00 |
| | Eumulgin B 1 | 0,25 |
| | Eumulgin B 2 | 0,25 |
| | Cutina MD | 2,00 |
| | Lanette O | 2,80 |
| | Myritol 318 | 5,00 |
| | Paraffinöl 65 cp | 3,00 |
| | Arosol | 0,80 |
| | Solbrol P | 0,10 |
| | p- Methoxyzimtsäureisooctylester | 3,00 |
| | p-Methoxyzimtsäureisoamylester | 3,00 |
| | Neo Heliopan, Typ BB | 1,50 |
| | | |
| B) | Wasser, dest. | 51,05 |
| | Carbopol 941 | 0,30 |
| | Natriumhydroxid, 10 %ig in Wasser | 2,45 |
| | 1,2-Propylenglykol | 2,00 |
| | Solbrol M | 0,20 |
| | Phenylen-1,4-bisbenzimidazol-5,5',7,7'-tetrasulfonsäure, eingesetzt als 20 %ige Lösung nach Neutralisation mit Natriumhydroxid | 20,00 |
| | entspricht Aktivsubstanz: 4,00 % | |
| | | |
| C) | Parfumöl | 0,30 |

### Herstellvorschrift:

- Teil A:: Bei ca. 75 bis 80°C aufschmelzen.
- Teil B:: Carbopol klumpenfrei im Wasser dispergieren, mit Natronlauge neutralisieren, die restlichen Inhaltsstoffe zugeben und auf ca. 95°C erhitzen.

Teil B unter Rühren zu Teil A geben und auf Raumtemperatur abrühren. Bei ca. 30°C Teil C zugeben. pH-Wert überprüfen (7,0-7,5).

### Beispiel 5

### Sonnenschutzlotion (O/W)

Von dem UV-Absorber Phenylen-1,4-bis-benzimidazol-5,5',7,7'-tetrasulfonsäure-bis-natriumsalz wurde eine 20 %ige Vorlösung, neutralisiert mit Natriumhydroxid, hergestellt Von dieser Lösung wurden 15 % eingesetzt, was einem Aktivgehalt von 3,00 % UV-A-Absorber in der Endformulierung entspricht.

| | Bestandteile | % |
|---|---|---|
| A) | Arlacel165 | 3,00 |
| | Eumulgin B 2 | 1,00 |
| | Lanette O | 2,00 |
| | Myritol 318 | 4,00 |
| | Cetiol OE | 6,00 |
| | Betone Gel MIO und Quaternium-18 Hectorite | (3,00) |
| | Phenonip | 0,20 |
| | Cutina CBS | 2,00 |
| | p-Methoxyzimtsäureisooctylester | 7,00 |
| | 4-Methylbenzyliden-campher | 1,00 |
| | Zinkoxid neutral H& R | 5,00 |
| | | |
| B) | Wasser, dest. | 45,90 |
| | Veegum Ultra | 1,00 |
| | Natrosol 250 HHR | 0,30 |
| | Glycerin 85 % | 3,00 |
| | Phenonip | 0,30 |
| | Phenylen-1,4-bisbenzimidazol-5,5',7,7'-tetrasulfonsäure, eingesetzt als 20 %ige Lösung nach Neutralisation mit Natriumhydroxid | 15,00 |
| | entspricht Aktivsubstanz: 3,00 % | |
| | | |
| C) | Parfumöl | 0,30 |

### Herstellvorschrift:

- Teil A:: Bei ca. 80°C aufschmelzen, dann Zinkoxid neutral sorgfältig dispergieren.
- Teil B:: Ohne Veegum und Natrosol auf ca. 90°C erhitzen, dann Veegum und Natrosol dispergieren. Teil B unter Rühren zu Teil A geben Auf Raumtemperatur abrühren.
- Teil C:: Bei 30°C Teil C zugeben und anschließend homogenisieren. pH-Wert überprüfen (7,0-7,5).

### Beispiel 6

### Sonnenschutzcreme (O/W)

Von dem UV-A-Absorber Phenylen-1,4-bis-benzimldazol-5,5',7,7'-tetrasulfonsäure-bis-natriumsalz wurde eine 20 %ige Vorlösung, neutralisiert mit Natriumhydroxid, hergestellt. Von dieser Vorlösung wurden 20 % eingesetzt, was einem Aktivgehalt von 4,00 % UV-A-Absorber in der Endformulierung entspricht.

| | Bestandteile | % |
|---|---|---|
| A) | Tegin M | 3,00 |
| | Tagat S | 2,30 |
| | Lanette O | 3,50 |
| | Paraffinöl 65 cp | 4,00 |
| | Arlamol HD | 3,50 |
| | Cetiol MM | 2,00 |
| | Phenonip | 0,20 |
| | p-Methoxyzimtsäureisooctylester | 4,00 |
| | p-Methoxyzimtsäureisoamylester | 4,00 |
| | | |
| B) | Wasser, dest. | 16,85 |
| | Phenonip | 0,20 |
| | Neo Heliopan, Typ Hydro, eingesetzt als 15 %ige Lösung nach Neutralisation mit Natriumhydroxid / Phenylbenzimidazolsulfonsäure | 6,70 |
| | entspricht Aktivsubstanz: 1,00 % | |
| | Phenylen-1,4-bisbenzimidazol-5,5',7,7'-tetrasulfonsäure, eingesetzt als 20 %ige Lösung nach Neutralisation mit Natriumhydroxid | 20,00 |
| | entspricht Aktivsubstanz: 4,00 % | |
| | 1,2-Propylenglykol | 2,00 |
| | | |
| C) | Wasser, dest. | 25,00 |
| | Carbopol 940 | 0,40 |
| | Natriumhydroxid, 10%ig in Wasser | 2,05 |
| | | |
| D) | Parfümöl | 0,30 |

### Herstellvorschrift:

- Teil A:: Bei ca. 80°C aufschmelzen.
- Teil B:: Auf ca. 90°C erhitzen. Teil B unter Rühren zu Teil A geben. Auf Raumtemperatur abrühren.
- Teil C:: Carbopol klumpenfrei in Wasser dispergieren, mit Natriumhydroxid-Lösung zu einem Gel neutralisieren, bei ca. 60°C zu Teil A/B geben.
- Teil D:: Bei ca. 30°C die Emulsion parfümieren. pH-Wert kontrollieren (7,0-7,5).

### Beispiel 7

### Sonnenschutzcreme (O/W)

Von dem UV-A-Absorber Phenylen-1,4-bis-benzimidazol-5,5',7,7'-tetrasulfonsäure-bis-natriumsalz wurde eine 20%ige Vorlösung, neutralisiert mit Natriumhydroxid, hergestellt. Von dieser Vorlösung wurden 25 % eingesetzt, was einem Aktivgehalt von 5,00 % UV-A-Absorber in der Endformulierung entspricht

| | Bestandteile | % |
|---|---|---|
| A) | Cutina FS 45 | 2,00 |
| | Eumulgin B 1 | 0,25 |
| | Eumulgin B2 | 0,25 |
| | Cutina MD | 2,00 |
| | Lanette O | 3,00 |
| | Myritol 318 | 5,00 |
| | Cetiol SN | 3,00 |
| | Arosol | 0,80 |
| | Solbrol P | 0,10 |
| | p-Methoxyzimtsäureisoamylester | 5,00 |
| | p-Methoxyzimtsäureisooctylester | 3,00 |
| | 4-Methylbenzyliden-campher | 1,00 |
| | Octylsalicylat | 3,00 |
| | Butylmethoxydibenzoylmethan | 1,00 |
| | | |
| B) | Wasser dest. | 40,90 |
| | Carbopol 940 | 0,40 |
| | Natriumhydroxid, 10%ig in Wasser | 1,80 |
| | 1,2-Propylenglykol | 2,00 |
| | Solbrol M | 0,20 |
| | Phenylen-1,4-bisbenzimidazol-5,5',7,7'-tetrasulfonsäure, eingesetzt als 20%ige Lösung nach Neutralisation mit Natriumhydroxid | 25,00 |
| | entspricht Aktivsubstanz: 5,00 % | |
| | | |
| C) | Parfumöl | 0,30 |

### Herstellvorschrift:

- Teil A:: Bei ca. 80°C aufschmelzen.
- Teil B:: Carbopol klumpenfrei in Wasser dispergieren, restliche Bestandteile zugeben und auf ca. 90°C erhitzen. Dann Teil B unter Rühren zu Teil A geben. Auf Raumtemperatur abrühren.
- Teil C:: Die Emulsion bei ca. 30°C parfümieren. pH-Wert kontrollieren (7,0-7,5).

### Beispiel 8

### Sonnenschutzmilch (W/O)

Von dem UV-A-Absorber Phenylen-1,4-bis-benzimidazol-5,5',7,7'-tetrasulfonsäure-bis-natriumsalz wurde eine 30%ige Vorlösung, neutralisiert mit Triethanolamin, hergestellt Von dieser Vorlösung wurden 6,7 % eingesetzt, was einem Aktivgehalt von 2,00 % UV-A-Absorber in der Endformulierung entspricht

| | Bestandteile | % |
|---|---|---|
| A) | Lameform TGI | 4,00 |
| | Dehymuls HR E 7 | 4,00 |
| | Cetiol S | 12,00 |
| | Paraffinöl 65 cp | 8,50 |
| | Permulgin 3220 | 1,00 |
| | p-Methoxyzimtsäureisooctylester | 5,00 |
| | p-Methoxyzimtsäureisoamylester | 5,00 |
| | Isopropyldibenzoylmethan | 1,00 |
| | | |
| B) | Wasser, dest. | 49,65 |
| | 1,2-Propylenglykol | 2,00 |
| | Triethanolamin | 0,35 |
| | Phenonip | 0,50 |
| | Phenylen-1,4-bisbenzimidazol-5,5',7,7'-tetrasulfonsäure, eingesetzt als 30%ige Lösung nach Neutralisation mit Triethanolamin | 6,70 |
| | entspricht Aktivsubstanz: 2,00 % | |
| | | |
| C) | Parfumöl | 0,30 |

### Herstellvorschrift:

- Teil A:: Bei ca. 80°C aufschmelzen.
- Teil B:: Bestandteile zusammengeben. pH-Wert der Wasserphase überprüfen (7,0-7,5). Auf ca. 90°C erhitzen. Teil B langsam unter Rühren zu Teil A geben. Auf Raumtemperatur abrühren.
- Teil C:: Die Emulsion bei ca. 35°C parfümieren, anschließend homogenisieren.

### Beispiel 9

### Sonnenschutzlotion (W/O)

Von dem UV-A-Absorber Phenylen-1,4-bis-benzimidazol-5,5',7,7'-tetrasulfonsäure-bis-natriumsalz wurde eine 30%ige Vorlösung, neutralisiert mit Triethanolamin, hergestellt. Von dieser Vorlösung wurden 20 % eingesetzt, was einem Aktivgehalt von 3,00 % UV-A-Absorber in der Endformulierung entspricht.

| | Bestandteile | % |
|---|---|---|
| A) | Elfacos E 200 | 3,00 |
| | Elfacos ST 9 | 1,00 |
| | Elfacos C 26 | 1,00 |
| | Paraffinöl 65 cp | 6,00 |
| | Isopropyldiisostearat | 7,00 |
| | p-Methoxyzimtsäureisooctylester | 7,00 |
| | Octylsalicylat | 5,00 |
| | Tioveil MOTG, 40%ige Dispersion | 12,50 |
| | | |
| B) | Wasser, dest. | 43,40 |
| | Trilon B fl. | 0,30 |
| | Glycerin 86 % | 3,00 |
| | Phenonip | 0,50 |
| | Phenylen-1,4-bisbenzimidazol-5,5',7,7'-tetrasulfonsäure, eingesetzt als 30%ige Lösung nach Neutralisation mit Triethanol-amin | 10,00 |
| | entspricht Aktivsubstanz: 3,00 | |
| | | |
| C) | Parfumöl | 0,30 |

### Herstellvorschrift:

- Teil A:: Bei ca. 80°C aufschmelzen und gut verrühren.
- Teil B:: Auf ca. 90°C erhitzen. Teil B unter Rühren zu Teil A geben. Auf Raumtemperatur abrühren.
- Teil C:: Bei ca. 30°C parfümieren, anschließend homogenisieren.

### Beispiel 10

### Sonnenschutzcreme (W/O)

Von dem UV-A-Absorber Phenylen-1,4-bis-benzimidazol-5,5',7,7'-tetrasulfonsäure-bis-natriumsalz wu rde eine 30%ige Vorlösung, neutralisiert mitTriethanolamin, hergestellt. Von dieser Vorlösung wurden 10% eingesetzt, was einem Aktivgehalt von 3,00 % UV-A-Absorber in der Endformulierung entspricht.

| | Bestandteile | % |
|---|---|---|
| A) | Protegin WX | 23,00 |
| | Alugel 30 DF . | 0,50 |
| | Paraffinöl 34 cp | 3,00 |
| | Solbrol R | 0,05 |
| | p-Methoxyzimtsäureisooctylester | 5,00 |
| | Neo Heliopan, Typ 303 | 7,00 |
| | Menthylanthranilat | 4,00 |
| | Eusolex 6007 | 4,00 |
| B) | Wasser, dest. | 37,30 |
| | Solbrol® M | 0,15 |
| | Magnesiumsulfat | 0,40 |
| | Sorbit 70 % | 5,00 |
| | Phenylen-1,4-bisbenzimidazol-5,5',7,7'-tetrasulfonsäure, eingesetzt als 30%ige Lösung nach Neutralisation mit Triethanolamin | 10,00 |
| | entspricht Aktivsubstanz: 3,00 | |
| | | |
| C) | Parfümöl | 0,30 |

### Herstellvorschrift:

- Teil A:: Alugel unter Erhitzen in Paraffinöl lösen. Die restlichen Bestandteile zugeben und bei ca. 80°C aufschmelzen.
- Teil B:: Auf ca. 90°C erhitzen. Teil B unter Rühren zu Teil A geben und auf Raumtemperatur abrühren.
- Teil C:: Die Creme bei ca. 30°C parfümieren und anschließend homogenisieren.

### Beispiel 11

### Sonnenschutzlotion (W/O)

Von dem UV-A-Absorber Phenylen-1,4-bis-benzimidazol-5,5',7,7'-tetrasulfonsäure-bis-natriumsalz wurde eine 30%ige Vorlösung, neutralisiert mit Triethanolamin, hergestellt. Von dieser Vorlösung wurden 10% eingesetzt, was einem Aktivgehalt von 3,00 % UV-A-Absorber in der Endformulierung entspricht.

| | Bestandteile | % |
|---|---|---|
| A) | Arlacel581 | 3,50 |
| | Arlatone T | 0,75 |
| | Cetiol S | 12,00 |
| | Paraffinöl 34 cp | 5,50 |
| | Permulgin 3220 | 1,00 |
| | p-Methoxyzimtsäureisooctylester | 5,00 |
| | p-Methoxyzimtsäureisoamylester | 5,00 |
| | Uvinul T 150 | 1,00 |
| B) | Wasser, dest. | 53,20 |
| | 1,2-Propylenglykol | 2,00 |
| | Triethanolamin | 0,35 |
| | Phenonip | 0,40 |
| | Phenylen-1,4-bisbenzimidazol-5,5',7,7'-tetrasulfonsäure, eingesetzt als 30%ige Lösung nach Neutralisation mit Triethanolamin | 10,00 |
| | entspricht Aktivsubstanz: 3,00 | |
| | | |
| C) | Parfumöl | 0,30 |

### Herstellvorschrift:

- Teil A:: Bei ca. 80°C aufschmelzen.
- Teil B:: Bestandteile zusammengeben. pH-Wert der Wasserphase überprüfen (7,0-7,5). Auf ca. 90°C erhitzen. Teil B langsam unter Rühren zu Teil A geben. Auf Raumtemperatur abrühren.
- Teil C:: Die Emulsion bei ca. 35°C parfümieren, anschließend homogenisieren.

### Beispiel 12

### Lippenschutzcreme (W/O)

Von dem UV-A-Absorber Phenylen-1,4-bis-benzimidazol-5,5',7,7'-tetrasulfonsäure-bis-natriumsalz wurde eine 30%ige Vorlösung, neutralisiert mitTriethanolamin, hergestellt. Von dieser Vorlösung wurden 10% eingesetzt, was einem Aktivgehalt von 3,00 % UV-A-Absorber in der Endformulierung entspricht.

| | Bestandteile | % |
|---|---|---|
| A) | Protegin WX | 23,00 |
| | Alugel 30 DF | 0,50 |
| | Paraffinöl 34 cp | 5,00 |
| | Cetiol V | 3,00 |
| | Solbrol P | 0,05 |
| | p-Methoxyzimtsäureisooctylester | 8,00 |
| | Butylmethoxydibenzoylmethan | 1,00 |
| | | |
| B) | Wasser, dest. | 43,30 |
| | Solbrol M | 0,15 |
| | Magnesiumsulfat | 0,40 |
| | Sorbit 70 % | 5,00 |
| | Phenylen-1,4-bisbenzimidazol-5,5',7,7'-tetrasulfonsäure, eingesetzt als 30%ige Lösung nach Neutralisation mit Triethanolamin | 10,00 |
| | entspricht Aktivsubstanz: 3,00 % | |
| | | |
| C) | Parfumöl | 0,30 |

### Herstellvorschrift:

- Teil A:: Alugel unter Erhitzen im Paraffinöl lösen. Die restlichen Bestandteile zugegeben und bei ca. 80°C aufschmelzen.
- Teil B:: Auf ca. 90°C erhitzen. Teil B unter Rühren zu Teil A geben und auf Raumtemperatur abrühren.
- Teil C:: Die Creme bei ca. 35°C parfümieren und anschließend homogenisieren.

### Beispiel 13

### Sonnenschutz-Hydrodispersionsgel, emulgatorfrei

Von dem UV-A-Absorber Phenylen-1,4-bis-benzimidazol-5,5',7,7'-tetrasulfonsäure-bis-natriumsalz wurde eine 30%ige Vorlösung, neutralisiert mit Triethanolamin, hergestellt. Von dieser Vorlösung wurden 10% eingesetzt, was einem Aktivgehalt von 3,00 % UV-A-Absorber in der Endformulierung entspricht.

| | Bestandteile | % |
|---|---|---|
| A) | Wasser, dest. | 75,00 |
| | Carbopol 1342 | 1,00 |
| | Triethanolamin | 1,20 |
| | | |
| B) | Phenylen-1,4-bisbenzimidazol-5,5',7,7'-tetrasulfonsäure, eingesetzt als 30%ige Lösung nach Neutralisation mit Triethanolamin | 10,00 |
| | entspricht Aktivsubstanz: 3,00 | |
| C) | p-Methoxyzimtsäureisooctylester | 3,00 |
| | p-Methoxyzimtsäureisoamylester | 3,00 |
| | Neo Heliopan, Typ BB | 1,00 |
| | Isopropylmyristat | 3,00 |
| | Baysilonöl PK 20 | 2,00 |
| | Phenonip | 0,50 |
| | Parfumöl | 0,30 |

### Herstellvorschrift:

- Teil A:: Carbopol klumpenfrei im Wasser dispergieren und mit Triethanolamin neutralisieren.
- Teil B:: UV-A-Absorber-Lösung in Teil A einführen.
- Teil C:: Neo Heliopan, Typ BB in Heliopan, Typ AV und Typ E 1000 lösen. Die restlichen Bestandteile zugeben und Teil C unter Rühren zu Teil A/B geben. Anschließend homogenisieren (Kolloid-Mühle) und den pH-Wert kontrollieren (7,0-7,5).

### Beispiel 14

### Sonnenschutzgel

Von dem UV-A-Absorber Phenylen-1,4-bis-benzimidazol-5,5',7,7'-tetrasulfonsäure-bis-natriumsalz wurde eine 30%ige Vorlösung, neutralisiert mit Triethanolamin, hergestellt. Von dieser Vorlösung wurden 13,3 % eingesetzt, was einem Aktivgehalt von 4,00 % UV-A-Absorber in der Endformulierung entspricht.

| | Bestandteile | % |
|---|---|---|
| A) | Ethylalkohol | 5,00 |
| | Wasser, dest. | 55,30 |
| | 1,2-Propylenglykol | 5,00 |
| | D-Panthenol | 0,50 |
| | Carbopol ETD 2001 | 1,10 |
| | | |
| B) | Wasser | 5,00 |
| | Triethanolamin | 2,30 |
| | | |
| C) | Neo Heliopan, Typ Hydro, eingesetzt als 30%ige Lösung nach Neutralisation mit Triethanolamin / Phenylbenzimidazolsulfonsäure | 10,00 |
| | entspricht Aktivsubstanz: 3,00 | |
| | Phenylen-1,4-bisbenzimidazol-5,5',7,7'-tetrasulfonsäure, eingesetzt als 30%ige Lösung nach Neutralisation mit Triethanol-amin | 13,30 |
| | entspricht Aktivsubstanz: 4,00 | |
| | | |
| D) | Cremophor NP 14 | 1,20 |
| | Parfumöl | 0,30 |

### Herstellvorschrift:

- Teil A:: Bestandteile im Wasser lösen, Carbopol klumpenfrei dispergieren.
- Teil B:: Triethanolamin im Wasser lösen und Teil B unter Rühren zu Teil A geben.
- Teil-C:: Die Lichtschutzfilter-Lösungen unter Rühren zum Gel Teil A/B geben.
- Teil D:: Parfumöl mit Cremophor vermischen und unterrühren und pH-Wert prüfen (7,0-7,5).

### Beispiel 15

### Sonnenschutz-Spray, Non-aerosol

Von dem UV-A-Absorber Phenylen-1,4-bis-benzimidazol-5,5',7,7'-tetrasulfonsäure-bis-natriumsalz wurde eine 30%ige Vorlösung, neutralisiert mit Triethanolamin, hergestellt. Von dieser Vorlösung wurden 23,35 % eingesetzt, was einem Aktivgehalt von 7,00 % UV-A-Absorber in der Endformulierung entspricht.

| | Bestandteile | % |
|---|---|---|
| A) | Ethylalkohol | 30,00 |
| | Parfumöl | 0,10 |
| | | |
| B) | Wasser, des | 39,80 |
| | Neo Heliopan®, Typ Hydro, eingesetzt als 15%ige Lösung nach Neutralisation mit Natriumhydroxid / Phenylbenzimidazolsulfonsäure | 6,75 |
| | entspricht Aktivsubstanz: 1,00 % | |
| | Phenylen-1,4-bisbenzimidazol-5,5',7,7'-tetrasulfonsäure, eingesetzt als 30%ige Lösung nach Neutralisation mit Triethanol-amin | 23,35 |
| | entspricht Aktivsubstanz: 7,00 % | |

### Herstellvorschrift:

- Teil A:: Parfumöl im Alkohol lösen.
- Teil B:: Bestandteile vermischen. Teil B zu Teil A geben und verrühren.

### Beispiel 16

### Haar-Shampoo mit Sonnenschutz

Von dem UV-A-Absorber Phenylen-1,4-bis-benzimidazol-5,5',7,7'-tetrasulfonsäure-bis-natriumsalz wurde eine 30%ige Vorlösung, neutralisiert mit Triethanolamin, hergestellt. Von dieser Vorlösung wurden 3,35 % eingesetzt, was einem Aktivgehalt von 1,00 % UV-A-Absorber in der Endformulierung entspricht.

| | Bestandteile | % |
|---|---|---|
| A) | Genapol LRO fl. | 18,00 |
| | Texapon MG 3 | 36,00 |
| | Lamepon S | 6,00 |
| | p-Methoxyzimtsäureisoamylester | 1,00 |
| | Parfumöl | 0,60 |
| B) | Wasser dest. | 34,60 |
| | Euxyl K 100 | 0,15 |
| | Phenylen-1,4-bisbenzimidazol-5,5',7,7'-tetrasulfonsäure, eingesetzt als 30%ige Lösung nach Neutralisation mit Triethanol-amin | 3,35 |
| | entspricht Aktivsubstanz: 1,00 % | |
| | Natriumhydroxid, 10%ig in Wasser | 0,10 |
| | Natriumchlorid | 0,20 |

### Herstellvorschrift:

- Teil A:: Bestandteile vermischen.
- Teil B:: Bestandteile im Wasser lösen. Teil B unter Rühren zu Teil A geben. Rühren bis homogenes Produkt entstanden ist und pH-Wert überprüfen (7,0-7,5).

### Beispiel 17

### Haargel mit Sonnenschutz

Von dem UV-A-Absorber Phenylen-1,4-bis-benzimidazol-5,5',7,7'-tetrasulfonsäure-bis-natriumsalz wurde eine 30%ige Vorlösung, neutralisiert mit Triethanolamin, hergestellt. Von dieser Vorlösung wurden 10% eingesetzt, was einem Aktivgehalt von 3,00 % UV-A-Absorber in der Endformulierung entspricht.

| | Bestandteile | % |
|---|---|---|
| A) | Wasser, dest. | 25,60 |
| | Glycerin 86 % | 26,00 |
| | Carbopol ETD 2001 | 1,50 |
| | | |
| B) | Wasser dest. | 3,00 |
| | Triethanolamin | 3,20 |
| | | |
| C) | Neo Heliopan®, Typ Hydro, eingesetzt als 30%ige Lösung nach Neutralisation mit Triethanolamin / Phenylbenzimidazolsulfonsäure | 6,70 |
| | entspricht Aktivsubstanz: 2,00 % | |
| | Phenylen-1,4-bisbenzimidazol-5,5',7,7'-tetrasulfonsäure, eingesetzt als 30%ige Lösung nach Neutralisation mit Triethanol-amin | 10,00 |
| | entspricht Aktivsubstanz: 3,00 % | |
| | Uvinul MS 40 | 1,50 |
| | Wasser, dest. | 20,00 |
| | | |
| D) | Mulsifan RT 203/80 | 1,20 |
| | Parfumöl | 0,30 |

### Herstellvorschrift:

- Teil A:: Bestandteile im Wasser lösen und Carbopol klumpenfrei dispergieren.
- Teil B:: Triethanolamin im Wasser lösen. Teil B unter Rühren zu Teil A geben.
- Teil C:: Bestandteile vermischen und unter Rühren in das Gel Teil A/B geben.
- Teil D:: arfumöl mit Mulsifan vermischen, in Teil A/B/C unterrühren und pH-Wert überprüfen (7,0-7,5).

### Beispiel 18

### Leave-on-Haarkur, transparent, mit Sonnenschutz

Von dem UV-A-Absorber Phenylen-1,4-bis-benzimidazol-5,5',7,7'-tetrasulfonsäure-bis-natriumsalz wurde eine 30%ige Vorlösung, neutralisiert mit Triethanolamin, hergestellt. Von dieser Vorlösung wurden 3,35 % eingesetzt, was einem Aktivgehalt von 1,00 % UV-A-Absorber in der Endformulierung entspricht.

| | Bestandteile | % |
|---|---|---|
| A) | Wasser, dest. | 81,50 |
| | Natrosol 250 HHR | 0,70 |
| | Ethylalkohol | 5,00 |
| | Uvinul P 25 | 5,00 |
| | Phenylen-1,4-bisbenzimidazol-5,5',7,7'-tetrasulfonsäure, eingesetzt als 30 %ige Lösung nach Neutralisation mit Triethanolamin | 3,35 |
| | entspricht Aktivsubstanz: 1,00 % | |
| | | |
| B) | Nutrilan L | 2,00 |
| | Dehyquart A | 0,20 |
| | Phenonip | 0,50 |
| | Triethanolamin | 0,25 |
| | | |
| C) | Mulsifan RT 203/80 | 1,20 |
| | Parfumöl | 0,30 |

### Herstellvorschrift:

- Teil A:: Wasser auf ca. 85°C erhitzen. Natrosol einstreuen und unter starkem Rühren auf Raumtemperatur abkühlen. Restliche Bestandteile zugeben.
- Teil B:: In Teil A einwiegen und verrühren.
- Teil C:: Mulsifan mit Parfumöl abmischen, in Teil A/B geben, gut verrühren und den pH-Wert kontrollieren (7,0-7,5).

### Verwendete Komponenten:

| | |
|---|---|
| Alugel 30 DF | Aluminiumdistearat, Lieferant 21 |
| Arlacel® 581 | Glycerinmono- und -distearat und Polyethylenglykolstearat, Lieferant 4 |
| Ariacel®165 | Glycerinstearat/Polyethylenglykol (MG 100)-stearat-Mischung, Lieferant 4 |
| Arlamol® HD | Isohexadecan, Lieferant 4 |
| Arlatone® T | Oleat des ethoxylierten Sorbits, Lieferant 5 |
| Arosol® | Phenoxyethanol, Lieferant 1 |
| Baysilone® Fluid PK 20 | Siliconöl, Lieferant 5 |
| Bentone® Gel MIO | Mineralöl, Lieferant 13 |
| Carbopol® 940 | Polyacrylsäure, Lieferant 2 |
| Carbopol® 941 | Polyacrylsäure, Lieferant 2 |
| Carbopol® 1342 | Polyacrylat, Lieferant 2 |
| Carbopol® ETD 2001 | Acrylsäure-Copolymerisat, Lieferant 2 |
| Cetiol® MM | Myristyl-myristat, Lieferant 3 |
| Cetiol® S | Dioctylcyclohexan, Lieferant 3 |
| Cetiol® V | Decyl-oleat, Lieferant 3 |
| Cetiol® OE | Dicaprylether, Lieferant 3 |
| Cetiol® SN | Cetyl-/Stearyl-isononanoat, Lieferant 3 |
| Cremophor® NP 14 | mit 14 Mol Ethylenoxid verethertes Nonylphenol, Lieferant 6 |
| Cutina® CBS | Glycerinstearat, Cetyl-/Stearylalkohol, Cetylpalmitat, Kokosnußglyceride, Lieferant 3 |
| Cutina® FS 45 | Palmitin-/Stearinsäure-Mischung, Lieferant 3 |
| Cutina® MD | Glycerinstearat, Lieferant 3 |
| Dehymuls® HR E 7 | ethoxyliertes hydriertes Ricinusöl, Lieferant 3 |
| Dehyquart® A | Cetyltrimethylammoniumchlorid, Lieferant 3 |
| Elfacos® C 26 | Hydroxyoctacosanyl-hydroxystearat, Lieferant 19 |
| Elfacos® E 200 | Ethylenglykol/Dodecylglykol-Polyether mit Methoxy-Endgruppen, Lieferant 19 |
| Elfacos® ST 9 | Ethylenglykol/Dodecylglykol-Polyether, Lieferant 19 |
| Eumulgin® B 1 | Cetyl-/Stearylalkohol, verethert mit 12 Mol Ethylenoxid, Lieferant 3 |
| Eumulgin® B 2 | Cetyl-/Stearylalkohol, verethert mit 20 Mol Ethylenoxid, Lieferant 3 |
| Eusolex® 6007 | N,N-Dimethyl-p-aminobenzoesäureoctylester, Lieferant 17 |
| Euxyl K 100 | 5-Chlor-2-methyl-4-isothiazolin-3-on, 2-Methyl-4-isothiazolin-3-on und Benzylalkohol, Lieferant 13 |
| Genapol® LRO fl. | Natriumlaurylsulfat, Lieferant 12 |
| Heliopan®, Typ AV | p-Methoxyzimtsäureisooctylester, Lieferant 1 |
| Heliopan®, Typ E 1000 | p-Methoxyzimtsäureisoamylester, Lieferant 1 |
| Lameform® TGI | Triglycerindiisostearat, Lieferant 3 |
| Lamepon® S | Eiweiß/Kokosfettsäure-Kondensat, Kaliumsalz, Lieferant 3 |
| Lanette® O | Cetyl-/Stearylalkohol-Mischung, Lieferant 3 |
| Mulsifan RT 203/80 | Fettalkoholpolyglykolether, Lieferant 9 |
| Myritol® 318 | Capryl-/Caprin-triglycerid, Lieferant 3 |
| Natrosol® 250 HHR | Hydroxyethylcellulose, Lieferant 15 |
| Neo Heliopan®, Typ BB | 2-Hydroxy-4-methoxybenzophenon, Lieferant 1 |
| Neo Heliopan®, Typ Hydro | Phenylbenzimidazolsulfonsäure, Lieferant 1 |
| Neo Heliopan®, Typ 303 | α-Phenyl-β-cyan-zimtsäureisooctylester, Lieferant 1 |
| Nutrilan® L | Eiweißhydrolysat, Na-salz, Lieferant 3 |
| Permulgin | Wachs, Lieferant 18 |
| Phenonip® | Gemisch von p-Hydroxybenzoesäureestern, Lieferant 11 |
| Protegin® WX | Kombination nicht-ionoger Fettsäureester mehrwertiger Alkohole mit Wachsen und gereinigten gesättigten Kohlenwasserstoffen, Lieferant 10 |
| Quaternium-18 Hectorit | Propylencarbonat, Lieferant 13 |
| Solbrol® P | p-Hydroxybenzoesäurepropylester, Lieferant 5 |
| Solbrol® M | p-Hydroxybenzoesäuremethylester, Lieferant 5 |
| Tagat® S | Polyoxyethylenglycerinmonostearat, Lieferant 10 |
| Tegin® M | Glycerinstearat, Lieferant 10 |
| Texapon® MG 3 | Magnesiumlaurylsulfat/Dinatriumlaurylsulfosuccinat, Lieferant 3 |
| Tioveil® MOTG | 40 gew.-%-ige wäßrige Dispersion von Titandioxid, Lieferant 20 |
| Trilon B® fl. | Tetranatrium-ethylendiamintetraessigsäure, Lieferant 6 |
| Uvinul® MS 40 | p-Hydroxybenzophenon, Lieferant 6 |
| Uvinul® P 25 | Polyethylenglykolester der p-Aminobenzoesäure, Lieferant 6 |
| Uvinul®T 150 | Triazinyl-p-aminobenzoesäureisooctylester, Lieferant 6 |
| Veegum® Ultra | Magnesiumaluminiumsilikat Lieferant 14 |

### Lieferanten

- 1.: Haarmann & Reimer GmbH, Holzminden
- 2.: B.F. Goodrich Comp., Neuss
- 3.: Henkel KGaA, Düsseldorf
- 4.: ICI Speciality Chemicals, Frankfurt
- 5.: Bayer AG, Leverkusen
- 6.: BASF, Ludwigshafen
- 7.: Sutton Lab. Inc., Chatham, N.J., USA
- 8.: Gattefossé, Saint-Priest Cedex
- 9.: Zschimmer & Schwarz GmbH, Lahnstein
- 10.: Goldschmidt AG, Essen
- 11.: Nipa Lab. Ltd., Pontypridd Mid Glam, Wales, GB
- 12.: Hoechst AG, Frankfurt
- 13.: Schülke & Mayr GmbH, Norderstedt
- 14.: R.T. Vanderbilt Company Inc., Norwalk, USA
- 15.: Hercules Inc., Wilmington, Del, USA
- 16.: Givaudan-Roure GmbH, Genf
- 17.: E. Merck, Darmstadt
- 18.: Koster Keunen Holland BV, Bladel, NL
- 19.: Akzo Chemie GmbH, Düren
- 20.: Tioxide Chemicals Ltd., Billingham, Cleveland, GB
- 21.: Chemische Werke Bärlocher, München

## Patentansprüche

1. Kosmetische und dermatologische Zubereitungen, enthaltend Phenylen-1,4-bisbenzimidazol-5,5',7-trisulfonsäure bzw. deren Salze oder Phenylen-1,4-bisbenzimidazol-5,5',7,7'-tetrasulfonsäure bzw. deren Salze als UV-A-Absorber.

2. Kosmetische und dermatologische Zubereitungen nach Anspruch 1 enthaltend 0,5 bis 15 Gew.-% der Phenylen-1,4-bisbenzimidazolsulfonsäuren bzw. deren Salze bezogen auf das Gesamtgewicht der Zubereitung.

3. Verwendung von Phenylen-1,4-bisbenzimidazol-5,5',7-trisulfonsäure bzw. deren Salze oder Phenylen-1,4-bisbenzimidazol-5,5',7,7'-tetrasulfonsäure bzw. deren Salze als UV-A-Absorber in kosmetischen und dermatologischen Zubereitungen.

4. Phenylen-1,4-bisbenzimidazol-5,5',7-trisulfonsäure bzw. deren Salze.

5. Phenylen-1,4-bisbenzimidazol-5,5',7,7'-tetrasulfonsäure bzw. deren Salze.

6. Verfahren zur Herstellung von kosmetischen und dermatologische Zubereitungen nach Anspruch 1 oder 2, **dadurch gekennzeichnet, dass** die Phenylen-1,4-bisbenzimidazolsulfonsäuren und deren Salze durch Umsetzung von o-Phenylendiamin und Terephthalsäure in Chlorsulfonsäure bei Temperaturen von 20 bis 190°C hergestellt und mit für diese Anwendung üblicherweise verwendeten Bestandteilen verarbeitet werden.

## Claims

1. Cosmetic and dermatological preparations containing phenylene-1,4-bisbenzimidazole-5,5',7-trisulfonic acid or the salts thereof or phenylene-1,4-bisbenzimidazole-5,5',7,7'-tetrasulfonic acid or the salts thereof as UV-A absorbers.

2. Cosmetic and dermatological preparations according to claim 1 containing 0.5 to 15 wt.% of the phenylene-1,4-bisbenzimidazolesulfonic acids or the salts thereof relative to the total weight of the preparation.

3. Use of phenylene-1,4-bisbenzimidazole-5,5',7-trisulfonic acid or the salts thereof or phenylene-1,4-bisbenzimidazole-5,5',7,7'-tetrasulfonic acid or the salts thereof as UV-A absorbers in cosmetic and dermatological preparations.

4. Phenylene-1,4-bisbenzimidazole-5,5',7-trisulfonic acid or the salts thereof.

5. Phenylene-1,4-bisbenzimidazole-5,5',7,7'-tetrasulfonic acid or the salts thereof.

6. A process for the production of cosmetic and dermatological preparations according to claim 1 or 2, **characterised in that** the phenylene-1,4-bisbenzimidazolesulfonic acids and the salts thereof are produced by the reaction of o-phenylenediamine and terephthalic acid in chlorosulfonic acid at temperatures of 20 to 190°C and are processed using constituents conventionally used for this application.

## Revendications

1. Préparations cosmétiques et dermatologiques contenant de l'acide phénylène-1,4-bis-benzimidazol-5,5',7-trisulfonique ou ses sels ou de l'acide phénylène-1,4-bisbenzimidazol-5,5',7,7'-tétrasulfonique ou ses sels comme absorbeur d'UV-A.

2. Préparations cosmétiques et dermatologiques selon la revendication 1 contenant 0,5 à 15 % en masse des acides phénylène-1,4-bisbenzimidazolsulfonique ou de leurs sels par rapport à la masse totale de la préparation.

3. Utilisation de l'acide phénylène-1,4-bisbenzimidazol-5,5',7-trisulfonique ou de ses sels ou de l'acide phénylène-1,4-bisbenzimidazol-5,5',7,7'-tétrasulfonique ou de ses sels comme absorbeur d'UV-A dans des préparations cosmétiques et dermatologiques.

4. Acide phénylène-1,4-bisbenzimidazol-5,5',7-trisulfonique ou ses sels.

5. Acide phénylène-1,4-bisbenzimidazole-5,5',7,7'-tétrasulfonique ou ses sels.

6. Procédé de production de préparations cosmétiques et dermatologiques selon la revendication 1 ou 2, **caractérisé en ce que** les acides phénylène-1,4-bisbenzimidazolsulfoniques et leurs sels sont préparés par réaction de la o-phénylènediamine et de l'acide téréphtalique dans l'acide chlorosulfonique à des températures de 20 à 190°C et sont mis en oeuvre avec des constituants utilisés habituellement pour cette application.
